Europäisches Patentamt

European Patent Office

Office européen des brevets

(11) Numéro de publication: **0 262 123 B1**

# FASCICULE DE BREVET EUROPEEN

(45) Date de publication de fascicule du brevet: **01.04.92**  (51) Int. Cl.⁵: **A61K 49/04**

(21) Numéro de dépôt: **86902412.5**

(22) Date de dépôt: **07.04.86**

(86) Numéro de dépôt internationale :
**PCT/FR86/00116**

(87) Numéro de publication internationale :
**WO 87/06139 (22.10.87 87/23)**

(54) **NOUVELLES COMPOSITIONS UTILISABLES EN TOMO-DENSITOMETRIE.**

(43) Date de publication de la demande:
**06.04.88 Bulletin 88/14**

(45) Mention de la délivrance du brevet:
**01.04.92 Bulletin 92/14**

(84) Etats contractants désignés:
**AT BE CH DE FR GB IT LI LU NL SE**

(56) Documents cités:
**EP-A- 0 173 629
DE-A- 3 401 052**

(73) Titulaire: **DIETLIN, François
17, rue du Maréchal Foch
F-78110 Le Vésinet(FR)**

Titulaire: **HEITZ, Fernand
3, avenue des Marroniers
F-94120 Fontenay s/Bois(FR)**

(72) Inventeur: **DIETLIN, François
17, rue du Maréchal Foch
F-78110 Le Vésinet(FR)**
Inventeur: **HEITZ, Fernand
3, avenue des Marroniers
F-94120 Fontenay s/Bois(FR)**

(74) Mandataire: **Burtin, Jean-François
Cabinet GEFIB 55 Rue Boissonade
F-75014 Paris(FR)**

Rank Xerox (UK) Business Services

## Description

La technique de tomo-densitométrie est une méthode d'exploration et d'imagerie du corps humain permettant entre autres la mise en évidence des organes pleins.

L'étude de l'abdomen en tomo-densitométrie nécessite l'emploi de produits de contraste pour l'identification des éléments anatomiques.

Ainsi, grâce à l'injection endo-veineuse de produits iodés hydrosolubles (dits angio-scan), on a mis en évidence les vaisseaux, de même que le parenchyme des organes pleins (foie, rein, rate..).

Les organes creux sont mal identifiables malgré l'emploi de produits iodés fortement dilués par voie orale. Leur densité optique d'une part, leur manque d'homogénéité d'autre part, font qu'ils ne répondent pas aux espoirs émis pour l'identification anatomique et le diagnostic d'éléments pathologiques.

On connaissait déjà l'emploi de telles solutions ou compositions à des fins de diagnostic bien différentes. La demande de brevet allemand 3.401.052 décrit déjà des compositions contenant des sels hydrosolubles de complexes métalliques et notamment de complexes métalliques dont le poids atomique est compris entre 45 et 63. Elles sont utilisées dans des méthodes de diagnostic comme les observations en RMN et par ultrason.

La même référence décrit en outre, des compositions renfermant des complexes métalliques de métaux lourds (poids atomique 139 à 209) qui trouvent un emploi dans le diagnostic par les rayons X.

En outre, le brevet européen 173.629 décrit la production de complexes anticorps -agent chelateur- ion métallique, solubles dans l'eau dont l'ion métallique est par exemple le scandium (PA 46), le fer (PA 52) ou le cobalt (PA 55).

Ces complexes sont utilisés soit pour des méthodes de traitement thérapeutique (destruction de cellules), soit pour des méthodes de diagnostic.

L'emploi de nouveaux produits de contraste constitués par des solutions concentrées à base de sels de métaux légers comme le calcium, le magnésium ou le zinc, répond aux critiques précédentes :
- ceux-ci remplissent l'ensemble de la lumière du tube digestif en moins d'une heure sans laisser de vide anatomique.
- ils soulignent parfaitement la paroi du tube digestif
- leur densité optique fait qu'ils ne peuvent être confondus avec un élément anatomique ou anatomo-pathologique du voisinage.
- ils peuvent être utilisés également pour la mise en évidence de la vessie et des voies génitales.

L'invention a donc pour objet, l'utilisation de compositions renfermant au moins un dérivé d'un métal dont le poids atomique est compris entre 24 et 66, soluble en milieu aqueux, en solution dans un véhicule aqueux à une concentration allant de 5 à 25 % dudit dérivé métallique, le véhicule aveux étant un de ceux appropriés pour l'administration par voie orale, rectale ou endocavitaire, pour l'obtention d'un agent diagnostic, employé pour les observations en tomo-densitométrie.

Parmi les métaux considérés, on citera plus particulièrement les dérivés du magnésium, les dérivés du calcium, les dérivés du manganèse, les dérivés du fer, les dérivés du zinc et les dérivés du cobalt.

Les sels utilisables sont des sels solubles dans l'eau permettant de réaliser des solutions renfermant de 5 à 25 % du dérivé métallique.

A cette fin, on utilisera de préférence un chlorure, un nitrate, un sulfate, un acétate et surtout un gluconate, un lactobionate, un glucoheptonate ou un gluconoglucoheptonate de métal.

On pourra ainsi avantageusement utiliser une solution de chlorure de magnésium, de nitrate de calcium, une solution de gluconoglucoheptonate de calcium, une solution de sulfate de zinc, une solution d'acétate de manganèse, ou une solution de formiate de magnésium.

Lorsqu'un métal possède plusieurs valences, le choix du dérivé se fera en fonction des critères de solubilité et de la stabilité du composé. On pourra, par exemple, utiliser indifféremment un dérivé ferreux ou ferrique, ou un dérivé manganeux ou un dérivé manganique.

Il peut également être avantageux d'utiliser dans le cas du cobalt ou du zinc, des complexes solubles comme par exemple ceux de l'acide éthylène-diaminotétra-acétique, ceux de l'acide éthylèneglycol bis (β-aminoéthyléther)-N,N, N',N'-tétra-acétique, de l'acide éthylènediamine-N,N'-diacétique-N,N'-di- α -propionique ou de l'acide éthylène-diamino-N,N'-diacétique-N,N'-di-β-propionique. Ces complexes peuvent plus particulièrement être constitués par des molécules mixtes renfermant deux ou quatre ions alcalins comme par exemple le complexte de l'acide éthylène-diaminetétra-acétique avec le zinc sous forme de sel tétrasodique.

L'emploi de complexes metalliques comporte l'avantage d'une saveur atténuée et d'une absence de toxicité.

Les solutions selon l'invention peuvent en outre être additionnées d'agent épaississant ou augmentant

2

la viscosité, d'agents édulcorants, d'agents aromatisants.

Les solutions sont destinées à être administrées par voie buccale sous forme de soluté, de sirop ou de potion ; ou par voie rectale sous forme de lavement ou par voie endocavitaire comme, par exemple, par instillation dans la vessie ou dans les voies génitales.

D'une manière préférée, on utilise une solution aqueuse à 5, 10 ou 15 % d'une sel de métal dont le poids atomique est compris entre 24 et 66, édulcorée par un agent édulcorant synthetique comme l'Aspartam ou un saccharinate comme celui de sodium ou de calcium.

On peut utiliser également des solutions renfermant un mélange de tels dérivés métalliques en proportions respectives variant de 1 à 99 %, comme par exemple une solution à parties égales d'un sel de magnésium et d'un sel de calcium. Les sels peuvent dériver du même anion ou, sauf incompatibilité chimique, de deux anions différents.

Pour l'examen des organes et, notamment, du type digestif, les compositions selon l'invention peuvent inclure, en outre, un médicament qui modifie la motricité et, de préférence, un medicament qui réduit ou supprime le péristaltisme intestinal. On citera, à cet égard, la trimébutine et ses sels, les sels de N-méthyl-scopolamine, les sels de N-butylhyoscine, les sels de prifinium, les sels de difémérine, les sels d'atropine, d'hyosciamine, de scopolamine, les sels de pentapripérium, le bromure de mépenzolate, le chlorhydrate de pipéridolate, le bromure de pinavérium, le chlorhydrate de mébévérine, le chlorhydrate d'octavérine ou le chlorhydrate d'oxyphencyclimine.

Le volume de solution qui doit être administré peut varier de 50 à 500 ml et de préférence de 100 à 300 ml en fonction de la voie d'administration.

Les solutions selon l'invention permettent donc d'obtenir des observations du tube digestif (portion haute ou portion basse, de la vessie et ces voies génitales) en tomo-densitomètre plus fine, plus contrastée et permettant de mieux apprécier les contours des organes creux.

Les exemples suivants illustrent l'invention. Ils ne la limitent en aucune façon.

EXEMPLE I :

| Soluté de gluconate de calcium | |
|---|---|
| . gluconate de calcium | 25 g |
| . carboxyméthylcellulose réticulée | 0,25 g |
| . Aspartam | 0,05 g |
| . arôme café | q.s. |
| . eau distillée | q.s.p 250 ml |

EXEMPLE II

| Soluté de glucono-glucoheptonate de calcium | |
|---|---|
| . glucono-glucoheptonate calcium | 30 g |
| . glycérol | 5 g |
| . Aspartam | 0,05 g |
| . arôme café | q.s. |
| . eau distillée | q.s.p 200 ml |

EXEMPLE III

| Soluté de gluconate ferreux | |
|---|---|
| . gluconate ferreux | 30 g |
| . hydroxypropylméthylcellulose | 0,10 g |
| . saccharinate de sodium | 0,15 g |
| . eau distillée | q.s.p 200 ml |

EXEMPLE IV

| Soluté d'acétate de magnésium | |
|---|---|
| . acétate de magnésium tétrahydraté | 37 g |
| . glycérol | 13 g |
| . saccharinate de sodium | 0,15 g |
| . eau distillée | q.s.p 200 ml |

EXEMPLE V

| Soluté de gluconate de magnésium | |
|---|---|
| . gluconate de magnésium (sel hémi-magnésien) | 46 g |
| . méthylcellulose | 4 g |
| . glycérol | 10 g |
| . Aspartam | 0,05 g |
| . eau distillée | q.s.p 200 ml |

EXEMPLE VI

| Soluté de lactobionate de calcium | |
|---|---|
| . lactobionate de calcium (sel hémi-calcique) | 27,5 g |
| . acétate de magnésium | 14 g |
| . eau distillée | q.s.p 200 ml |

EXEMPLE VII

| Soluté de cobalt | |
|---|---|
| . cobalto-éthylènediaminotétracémate disodique | 65 g |
| . carboxyméthylcellulose | 2,50 g |
| . glycérol | 12 g |
| . eau distillée | q.s.p 250 ml |

EXEMPLE VIII

4

| Soluté de gluconate de calcium | |
|---|---|
| . gluconate de calcium | 25 g |
| . carboxyméthylcellulose réticulée (sel de sodium) | 0,05 g |
| . bromure de prifinium | 0,05 g |
| . saccharinate de calcium | 0,15 g |
| . eau distillée | q.s.p 200 ml |

**Revendications**

1. Utilisation de compositions renfermant au moins un dérivé soluble en milieu aqueux, d'un métal dont le poids atomique est compris entre 24 et 66, en solution contenant de 5 à 25% dudit dérivé métallique dans un véhicule aqueux approprié pour l'administration par voie orale, rectale ou endocavitire, pour l'obtention d'un agent diagnostic employé pour les observations en tomo-densitométrie.

2. Utilisation selon la revendication 1°, dans laquelle le dérivé soluble en milieu aqueux est un sel soluble dans l'eau.

3. Utilisation selon la revendication 1° ou la revendication 2°, dans laquelle le métal est choisi parmi le magnésium, le calcium, le fer, le manganèse, le cobalt et le zinc.

4. Utilisation selon l'une des revendications 1 à 3, dans laquelle le dérivé métallique est choisi parmi les chlorures, les nitrates, les sulfates, les acétates, les gluconates, les lactobionates, les glucoheptonates et les gluconoglucoheptonates.

5. Utilisation selon l'une des revendications 1 à 3, dans laquelle le dérivé métallique est un complexe de l'acide éthylènediaminotétraacétique ou l'acide éthylènediamino-diacétique dipropionique avec le zinc ou le cobalt.

6. Utilisation selon l'une des revendications 1 à 5, caractérisée en ce qu'elle renferme, en outre, un principe actif qui modifie la motricité des organes.

7. Utilisation selon la revendication 6, dans laquelle le principe actif qui modifie la motricité des organes est le bromure de Prifinium.

8. Utilisation selon l'une des revendications 1 à 3, dans laquelle le principe actif est un mélange de 1 à 99% d'un dérivé de métal dont le poids atomique est compris entre 24 et 66 et de 99 à 1 % d'un dérivé d'un autre métal dont le poids atomique est compris entre 24 et 66.

**Claims**

1. Use of compositions containing at least a metallic derivative which is soluble in aqueous medium and the atomic weight of which lies between 24 and 66, in a solution containing from 5 to 25% of the said metallic derivative in an aqueous vehicle suitable for administration through the oral, rectal or endocavitary ways, for obtaining a diagnostic agent used for the observations in tomo-densitometry.

2. Use according to claim 1°, in which the derivative which is soluble in an aqueous medium, is a salt which is soluble in water.

3. Use according to claim 1° or claim 2° in which the metal is selected among magnesium, calcium, iron, manganese, cobalt and zinc.

4. Use according to any of claims 1° to 3°, in which the metallic derivative is selected among the chlorides, the nitrates, the sulphates, the acetates, the gluconates, the lactobionates, the glucoheptonates and the gluconoglucoheptonates.

5. Use according to any of claims 1 to 3 in which the metallic derivative, is a complex of ethylene diaminotetraacetic acid, or of ethylene diaminodiacetic dipropionic acid with zinc or cobalt.

6. Use according to any of claims 1 to 5 characterized in that it contains further another active ingredient which modifies the motility of the organs.

7. Use according to claim 6 in which the active ingredient which modifies the motility of the organs, is Prifinium bromide.

8. Use according to any of claims 1 to 3 in which the active ingredient is a mixture made of 1 to 99% of a metallic derivative the atomic weight of which lies between 24 and 66 and of 99 to 1% of a derivative of another metal the atomic weight of which lies between 24 and 66.

**Patentansprüche**

1. Verwendung von Zusamenzetzungen die, mindenstens eine in wassrige Medium lösliche Derivate eines Metalls dessen atomische Gewicht zwischen 24 and 66 liegt, enthalten, in einer Lösung die von 5 bis 25 % des genannten metallischen Derivates enthält, in einem wässrigen Vehikel das fur die Verabreichung durch orale, rektale oder endokavitare Weg geeignet ist, für die Herstellung eines fur die Untersuchungen in tomo-densitometrie verwendeten diagnostischen Mitells.

2. Verwendung nach Anspruch 1° worin der in wassrigen Medium losliche Derivate, ein ins Wasser lösliche Salz ist.

3. Verwendung nach Anspruch 1° oder Anspruch 2°, worin das Metal zwischen Magnesium, Kalzium, Eisen, Mangan, Kobalt und Zink gewählt ist.

4. Verwndung nach einer der Ansprüche 1° bis 3°, worin der metallischen Derivate zwischen Chloriden, Nitraten, Sulfaten, Azetaten, Glukonaten, Laktobionaten, Glukoheptonaten und Glukonoglukoheptonaten gewählt ist.

5. Verwendung nach einer der Ansprüche 1° bis 3°, worin der metallischen Derivate einen Komplex des Zinks oder des Kobalts mit Ethylendiaminotetraessig Saüre oder Ethylendiaminodiessig Saüre dipropionsaüre, ist.

6. Verwendung nach einer der Ansprüche 1° bis 5° dadurch gekennzeichnet dass sie weiterhin, eine Wirkstoff der die Bewegbarkeit der Organen verändert, enthält.

7. Verwendung nach Ansprüch 6 worin der Wirkstoff der die Bewegbarkeit der Organen verändert, Prifinium Bromid ist.

8. Verwendung nach einer der Ansprüche 1° bis 3°, worin der Wirkstoffe einer Mischung aus 1 bis 99% eines metallischen Derivaten dessen atomischen Gewicht zwischen 24 and 66 liegt und aus 99 bis 1% eines Derivates eines anderen Metalls, das atomische Gewicht dessen, zwischen 24 und 66 liegt.